(19) Europäisches Patentamt
European Patent Office
Office européen des brevets

(11) Veröffentlichungsnummer : **0 069 342**
**B1**

(12) # EUROPÄISCHE PATENTSCHRIFT

(45) Veröffentlichungstag der Patentschrift : **09.01.85**

(51) Int. Cl.[4] : **A 61 L 2/26**

(21) Anmeldenummer : **82105850.0**

(22) Anmeldetag : **01.07.82**

(54) **Medizinische Geräte aus Kunststoffen, die Indikatoren für Sterilisationsverfahren enthalten.**

(30) Priorität : **03.07.81 DE 3126275**

(43) Veröffentlichungstag der Anmeldung : **12.01.83 Patentblatt 83/02**

(45) Bekanntmachung des Hinweises auf die Patenterteilung : **09.01.85 Patentblatt 85/02**

(84) Benannte Vertragsstaaten : **AT BE CH DE FR GB IT LI LU NL SE**

(56) Entgegenhaltungen :
**FR-A- 2 252 619**
**GB-A- 920 689**
**US-A- 3 667 916**
**US-A- 4 028 118**

(73) Patentinhaber : **Intermedicat GmbH**
**Gerliswilstrasse 45**
**CH-6020 Emmenbrücke (CH)**

(72) Erfinder : **Herlitze, Gerhard**
**Binsdorfer Strasse 4**
**D-3507 Baunatal-Ginterhausen (DE)**

(74) Vertreter : **von Kreisler, Alek, Dipl.-Chem. et al**
**Deichmannhaus am Hauptbahnhof**
**D-5000 Köln 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 069 342 B1

**Beschreibung**

Die Erfindung betrifft medizinische Geräte aus Kunststoffen, insbesondere Einmalgeräte, die aus Polymeren bestehen, die Indikatoren für Sterilisationsbehandlungen enthalten.

Bei der Herstellung medizinischer und sanitärer Artikel wie Verbandstoffe, chirurgische Instrumente, Einmalartikel aus Kunststoffen zur Verwendung im medizinischen Bereich ist es notwendig, daß diese im sterilisierten keimfreien Zustand an den Verbraucher geliefert werden und daß er, ohne sie noch einmal sterilisieren zu müssen, diese steril aus ihren Verpackungen entnehmen und anwenden kann. Verschiedene Sterilisationsmethoden sind bekannt, die sich jedoch wegen der unterschiedlichen thermischen Belastbarkeit der einzelnen Werkstoffe nicht wahllos auf alle Gegenstände übertragen lassen. Bei einer « klassischen » Sterilisationsmethode werden die Gegenstände in einem Autoklaven bei 120 °C während 30-60 Minuten strömendem Wasserdampf ausgesetzt. Diese Dampfsterilisation ist einfach auszuführen, eignet sich aus ersichtlichen Gründen jedoch nur für solche Gegenstände und Materialien, die unter den Sterilisationsbedingungen ihre Zusammensetzung, ihre Form, allgemein gesagt, ihren Gebrauchswert beibehalten. Textilien oder metallische Geräte sind als Beispiele hierfür zu nennen.

In Fällen, wo wärmeempfindliche Produkte unter diesen Bedingungen nicht sterilisiert werden können, dies gilt z. B. für nur einmal zu verwendende Geräte aus Kunststoffen, die sich bei der Dampfsterilisation zur Unbrauchbarkeit verformen würden, müssen andere Sterilisationsverfahren herangezogen werden.

Kunststoffe, vor allem thermoplastische Kunststoffe, sind nicht ausschließlich, aber doch zum großen Teil bei Temperaturen über 100 °C nicht mehr formstabil, und gerade die preiswerten Standardkunststoffe wie Polyäthylen, Polystyrol, Polyvinylchlorid, Polymethylacrylate und Styrol-Copolymerisate werden zur Herstellung von medizinischen Einmalartikeln eingesetzt.

Alternative, auch im technischen Ablauf der Dampfsterilisation aus ökonomischen Gründen überlegene Sterilisationsmethoden sind die Sterilisation mit Äthylenoxid und die Sterilisation mit ernergiereicher Strahlung.

Im Hinblick auf die Qualitätskontrolle, die Produkt- und Verbrauchersicherheit ist es unerläßlich, daß einem Sterilisationsvorgang unterworfene Gegenstände im weiteren Verlaufe bis zum Verbraucher in der einen oder anderen Form als sterilisiert erkannt werden können. Dies kann einmal dadurch geschehen, daß auf Einzel- oder Sammelverpackungen ein schriftlicher Hinweis auf die erfolgte Sterilisation angebracht ist, der von dem interessierten und betroffenen Personenkreis gelesen werden kann.

Bei der Sterilisation mit energiereicher Strahlung, z. B. Co 60-Strahlung, ist es üblich, daß die Wirkung dieser Strahlung auf radiochrome Farbstoffe oder Pigmente genutzt wird, wobei aus den Farbstoffen durch Radiolyse anders gefärbte chemische Verbindungen entstehen. Zur Anzeige einer erfolgten Strahlensterilisation werden demgemäß z. B. Klebeetiketten in gelber Farbe auf Verpackungen angebracht, die nach der üblichen Bestrahlungsdosis von 2,5 Mrad nach rot verfärbt sind. In diesem Fall sind die visuell leicht identifizierbaren roten Etiketten der Beleg für eine erfolgte Sterilisierung.

Ein anderes übliches Verfahren geht von der Beobachtung aus, daß Halogen enthaltende Polymere wie Vinylchlorid- oder Vinylidenchloridpolymerisate oder niedrigmolekulare, nicht flüchtige halogenhaltige Substanzen wie chlorierte Paraffine unter dem Einfluß energiereicher, ionisierender Strahlung die entsprechende Halogensäure, z. B. Chlorwasserstoffsäure (HCl) bilden und dadurch die Farbe eines säureempfindlichen Farbstoffes, z. B. eines in der chemischen Analytik üblichen Titrationsindikators, verändert werden kann. Dementsprechend werden für strahlungsempfindliche, in der Farbe sich verändernde Indikatoren halogenhaltige und Halogenwasserstoff abspaltende Verbindungen mit einem auf Halogenwasserstoff abspaltende Verbindungen mit einem auf Halogenwasserstoffsäure unter Farbänderung reagierenden Farbstoff kombiniert.

Eine unter den Sterilisationsbedingungen sich ergebende Farbänderung wird ebenfalls bei der Dampfsterilisation mittels irreversibel sich ändernden thermochromen Farbstoffen und auch bei der Sterilisation mit Äthylenoxid werden chemisch ausgelöste Farbreaktionen als Sterilisationsindikator angewendet.

Als Beispiel für den Stand der Technik sei die DE-OS 29 29 582 genannt.

Die DE-OS 29 29 582 betrifft ein Verfahren zur Herstellung von sterilisierbaren Verpackungen, insbesondere für Gegenstände zur Anwendung im medizinischen Bereich, bei dem ein aus keim-, flüssigkeits- und gasdichtem Werkstoff bestehender Verpackungsteil mit einem aus keimdichtem, jedoch gas- und/oder dampfdurchlässigen Werkstoff bestehenden Verpackungsteil verbunden, z. B. verschweisst wird, und der gas- und/oder dampfdurchlässige Verpackungsteil vor oder nach der Verbindung mit dem undurchlässigen Verpackungsteil zumindest auf einer an der verschlossenen Verpackung von aussen sichtbaren Fläche mit einer Indikatorfarbe versehen wird, das dadurch gekennzeichnet ist, daß als Indikatorfarbe eine solche verwendet wird, die sich bei Berührung mit Feuchtigkeit bleibend verändert.

Bei industriell durchgeführten Sterilisationen medizinischer Einmalgeräte, die, wie erwähnt, vorwiegend aus thermolabilen Kunststoffen gefertigt sind, werden diese vorwiegend auf selbstklebenden Substraten aufgebrachten Indikatoren z. B. als Etiketten oder Klebebandabschnitte auf die Einzel- oder Sammelverpackung geklebt, wobei aus Kostengründen der Markierung der Sammelpackung der Vorzug gegeben wird. Aus den Sammelpackungen vereinzelte Packungen sind demzufolge nicht mehr als nach diesem jeweiligen Verfahren sterilisiert zu erkennen.

Es besteht daher ein Bedürfnis, medizinische Geräte und insbesondere medizinische Einmalgeräte und auch einzelne medizinische Geräte aus Sammelpackungen so zu kennzeichnen, daß eine an diesen Geräten durchgeführte Sterilisationsbehandlung einwandfrei und dauerhaft angezeigt wird.

Die Erfindung stellt sich daher die Aufgabe, medizinische Geräte zu schaffen, bei denen eine durch Wärmeeinwirkung, durch Einwirkung energiereicher Strahlung oder durch Einwirkung keimtötender gasförmiger Agentien durchgeführte Sterilisationsbehandlung dauerhaft und sicher angezeigt wird.

Diese Aufgabe wird durch die erfindungsgemäss zur Verfügung gestellten medizinischen Geräte gelöst.

Die Erfindung betrifft demnach medizinische Geräte aus Kunststoffen, insbesondere Einmalgeräte aus Kunststoffen, die dadurch gekennzeichnet sind, daß sie aus Polymeren bestehen, die Indikatoren für Sterilisationsbehandlungen enthalten. Im Prinzip können alle Indikatoren verwendet werden, die eine deutliche, unterscheidbare Farbänderung ergeben. Insbesondere sind die thermochromen oder radiochromen oder chemisch reagierenden Farbindikatoren geeignet.

Bevorzugt werden Indikatoren, die vor der Sterilisation farblos und nach der Sterilisation farbig erscheinen.

Weiterhin bevorzugt sind Indikatorsubstanzen, die inb Abhängigkeit von der Sterilisationsart einen unterschiedlichen Farbton ergeben und damit später einen Hinweis auf die angewandte Sterilisationsmethode zulassen.

Beispiele für Sterilisationsbehandlungen sind die üblichen Verfahren der Dampfsterilisarion, der Strahlensterilisation z. B. mit Kobalt-60 oder der Äthylenoxidsterilisation.

Erfindungsgemäss werden den zur Herstellung der aus Kunststoffen bestehenden Einmalgeräte verwendeten Werkstoffen, d. h. den polymeren Massen, Indikatoren zugegeben und diese als Zuschlagstoffe in die Werkstoffe eingearbeitet, so daß sie bei einem Sterilisationsvorgang durch die Einwirkung des sterilisierenden, die lebenden und vermehrungsfähigen Mikroorganismen abtötenden Agens eine Farbänderung erleiden. Damit wird auch die Farbe der damit ausgerüsteten Konstruktionsteile bleibend so verändert und je nach Art und Funktion des Konstruktionsteils bis zur oder auch bis nach der Anwendung erkennbar, daß es einer Sterilisation unterworfen war.

Aus toxikologischen Gründen wird man toxikologisch nicht geprüfte Substanzen bzw. deren Folgeprodukte nur in solchen Teilen als zulässig erachten, die mit dem Körper eines Patienten nicht in Kontakt kommen oder aus denen diese Reaktionsprodukte nicht extrahiert und in den Organismus gebracht werden können. Als Beispiele dafür wären vor der Verwendung zu entferndende Schutzkappen von Einmalgeräten oder Durchflußregler bei Infusions- oder Transfusionsgeräten zu nennen.

Radiochrome oder thermochrome oder chemisch reagierende Farbindikatoren sind allgemein bekannt.

Bekannt sind eine reichliche Zahl radiochromer Farbstoffe, die, auch in Abhängigkeit von der Größe der Strahlungsdosis, Farbänderungen unterliegen. Die üblicherweise zur oxidativen und chemischen Stabilisierung von Polyolefinen (Polyäthylen, Polypropylen) empfohlenen phenolischen Stabilisatoren (Antioxidation) der allgemeinen Formel

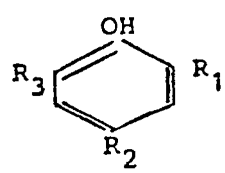

in der $R_1$, $R_2$ und $R_3$ Substituenten in 2-, 4- und 6-Stellung sind, unterliegen bei Einwirkung energiereicher Strahlung einer Reaktion, bei der durch Oxidation entstehende, im sichtbaren Teil des Spektrums absorbierende, chromophore aromatische Farbstoffmoleküle gebildet werden. Diese Farbänderung kann als Indiz für eine erfolgte Strahlensterilisation dienen.

Für die Anwendung unter Halogenwasserstoffabspaltung verwendbare Indikatoren sind prinzipiell alle chemischen Verbindungen, die beim Übergang vom basischen oder neutralen in den sauren-pH-Bereich einen deutlich erkennbaren Farbumschlag haben. Derartige Verbindungen sind nach der GB-PS 920 689 Dimethylgelb (p-dimethylamino-azo-benzol), Phenyl-2-azo-1-naphtylamin, Methylrot (p-dimethylamino-azo-benzolkarbonsäure), Congorot (pp'-diphenylen-bis-2-azo-naphtylamin-4-sulfonsäure) und Thymolblau (sulfonierter Triphenylmethanfarbstoff). Weitere nützliche Indikatorfarbstoffe sind auch in dem Katalog Reagenzien Diagnostica Chemikalien der E. Merck, Darmstadt, 1978, Teil 3, S. 24, angegeben.

Weiterhin sind Indikatoren im Sinne der Verfahrensweise für Äthylenoxid bekannt, die unter dessen Einwirkung und Reaktion sich farblich verändern. Das US-Patent 3 852 034 beschreibt die Verwendung von para-Rosanilinhydrochlorid in einem Puffersystem. Der Farbumschlag bei Einwirkung von Äthylenoxid erfolgt von rot nach blau. In der US-PS 4 094 642 wird 4-(4-Nitrobenzyl) pyridin im basischen Medium als Indikator angegeben. Als Beispiel für eine auf Dampf- und Äthylenoxidsterilisation ansprechende Chemikalie sei auf die US-PS 3 667 916 verwiesen, die von praktisch farblos nach dunkel umschlägt und aus Silbernitrat und einer konservierenden weiteren Verbindung besteht.

Die Erfindung wird nachstehend anhand von Beispielen erläutert.

Beispiel 1

Zu 25 kg eines handelsüblichen Polyethylens hoher Dichte (HDPE) werden 25 g β-(3,5-Ditert-butyl-4-hydroxiphenyl)-propionsäure-n-octadecylester als Indikator für die Strahlensterilisation gegeben und in einem Mischer geeigneter Bauart auf das Polyethylengranulat, evtl. unter Zusatz geringer Mengen eines Haftmittels, aufgetrommelt. Das so behandelte und vorbereitete Granulat, das den Indikator auf der Oberfläche verteilt enthält, wird in üblicher Weise auf einer Spritzgießmaschine im Spritzgießverfahren zu einem in der Medizin üblichen Verschlußteil für Kegelverbindungen verarbeitet. Bei der Sterilisation des verpackten Gegenstandes mit ionisierender Strahlung aus einer Cobalt-60-Quelle bei einer 2,5 Mrad-Dosis erfolgt ein Farbumschlag von farblos nach hellgelb.

Beispiel 2

Zu 25 kg eines handelsüblichen Propylenhomopolymerisats (PP) in Granulatform werden 20 g 2,4-Dimethyl-6-tert-butylphenol als Indikator für die Strahlensterilisation gegeben und in einem Mischvorgang, evtl. unter Zugabe einer geringen Menge eines Haftvermittlers wie Butylstearat, gleichmäßig auf der Granulatoberfläche verteilt. Im üblichen Spritzgießverfahren werden auf einer Spritzgießmaschine daraus Schutzkappen für Infusionsgeräte hergestellt und nach Montage der Schutzkappen die kompletten Geräte verpackt und in einer Sterilisationsanlage mit einer Cobalt-60-Quelle mit einer Dosis von 2,5 Mrad sterilisiert. Die Schutzkappen sind nach beendeter Sterilisation gelb gefärbt und erlauben somit eine Unterscheidung zu nicht sterilisierten Geräten.

Beispiel 3

Zur Herstellung eines in einem Urinsammelgerät verwendeten Rohres aus PVC-hart werden 25 kg verarbeitungsfertiges PVC-Pulvercompound mit 12 g Thymolblau intensiv gemischt und aus dieser Mischung in üblicher Weise auf einem Extruder ein Rohr mit einem Außendurchmesser von 5 mm extrudiert. Abschnitte des so erhaltenen Rohres werden als Überlaufrohr in die Sammelvorrichtung eingebaut und dieses Gerät nach Verpackung einer Strahlensterilisation mit einer 2,5 Mrad-Dosis unterworfen. Durch die aus dem PVC-hart-Polymeren in geringer Menge bei der Strahlensterilisation freigewordene Chlorwasserstoffsäure ist der Indikator von gelb nach rot umgeschlagen. Das rötlich gefärbte Rohr zeigt die erfolgte Sterilisation an.

Beispiel 4

25 kg Polystyrol in Granulatform werden mit 18,7 g m-Kresolpurpur in einem Mischer gut gemischt, bis der Indikatorfarbstoff gleichmäßig auf dem Granulat verteilt ist. Aus dem so vorbereiteten Granulat werden im Spritzgießverfahren Verbindungsstücke mit einem Außenkegel, die in medizinischen Einmalgeräten zur Herstellung von z. B. Schlauchleitungsverbindungen üblich sind, und mit den die Leitung darstellenden Schlauchabschnitten kombiniert. Die verpackten Gegenstände werden nach Feuchtigkeitskonditionierung einer Sterilisation mit einem Ethylenoxid-/Kohlendioxidgasgemisch unterworfen. Der Indikator ändert dabei seine Farbe von rot nach gelb und zeigt damit eine erfolgte Ethylenoxidbehandlung an.

Beispiel 5

Zur Herstellung einer einteiligen Schlauchklemme, die zur Unterbrechnung von Leitungswegen für medizinisch verwendete Infusionslösungen verwendet wird, werden 25 kg Methylmethacrylatpolymerisat (PMMA) mit 13,4 g Bromkresolgrün vermischt und im Spritzgießverfahren zu dem Formteil verarbeitet. Die dem medizinischen Gerät beigepackte Schlauchklemme wir mit diesem zusammen einer Ethylenoxidsterilisation unterworfen, wobei sich der Indikator von gelb nach blau ändert.

**Ansprüche**

1. Medizinische Geräte aus Kunstoffen, insbesondere Einmalgeräte, dadurch gekennzeichnet, daß sie aus Polymeren bestehen, die Indikatoren für Sterilisationsbehandlungen enthalten.
2. Medizinische Geräte nach Anspruch 1, dadurch gekennzeichnet, daß die Indikatoren der Sterilisationsbehandlung angepasst sind.
3. Medizinische Geräte nach Ansprüchen 1 und 2, dadurch gekennzeichnet, daß die Indikatoren vor der Sterilisation farblos sind.
4. Medizinische Geräte nach Ansprüchen 1-3, dadurch gekennzeichnet, daß die Indikatoren radiochrome Farbstoffe für die Strahlensterilisation sind.
5. Medizinische Geräte nach Ansprüchen 1-4, dadurch gekennzeichnet, daß die strahlungsempfindlichen Indikatoren halogenhaltige und Halogenwasserstoff abspaltende Verbindungen in Kombination

mit einem auf Halogenwasserstoffsäure unter Farbänderung reagierenden Farbstoff sind.

6. Medizinische Geräte nach Ansprüchen 1-3, dadurch gekennzeichnet, daß die Indikatoren thermochrome Farbstoffe für die Dampfsterilisation sind.

7. Medizinische Geräte nach Ansprüchen 1-3, dadurch gekennzeichnet, daß die Indikatoren auf Gassterilisation z. B. mit Äthylenoxid reagierende Farbstoffe sind.

**Claims**

1. Medical equipment made of synthetic materials, more specifically one-way equipment, characterized in that they consist of polymers containing indicators for sterilization treatments.

2. Medical equipment according to claim 1, characterized in that the indicators are adapted to the sterilization treatment.

3. Medical equipment according to claims 1 and 2, characterized in that the indicators are coulourless prior to the sterilization.

4. Medical equipment according to claims 1 to 3, characterized in that the indicators are radiochromic dyes for radiation sterilization.

5. Medical equipment according to claims 1 to 4, characterized in that the radiation-sensitive indicators are halogen-containing and hydrogen halide-yielding compounds in combination with a dye reacting with a hydrogen halide acid such as to change its color.

6. Medical equipment according to claims 1 to 3, characterized in that the indicators are thermochromic dyes for steam sterilization.

7. Medical equipment according to claims 1 to 3, characterized in that the indicators are dyes reactive to gas sterilization, e. g. dyes reacting with ethylene oxide.

**Revendications**

1. Articles médicaux en matières synthétiques, en particulier articles pour usage unique, caractérisés en ce qu'ils consistent en des polymères contenant des indicateurs pour des traitements de stérilisation.

2. Articles médicaux selon la revendication 1, caractérisés en ce que les indicateurs sont adaptés au traitement de stérilisation.

3. Articles médicaux selon les revendications 1 et 2, caractérisés en ce que les indicateurs sont incolores avant la stérilisation.

4. Articles médicaux selon les revendications 1 à 3, caractérisés en ce que les indicateurs sont des colorants radiochromes signalant la stérilisation par des rayonnements.

5. Articles médicaux selon les revendications 1 à 4, caractérisés en ce que les indicateurs sensibles à un rayonnement sont des composés contenant de l'halogène et cédant de l'halogénure d'hydrogène, en combinaison avec un colorant qui réagit, en changeant de couleur, avec l'hydracide halogéné.

6. Articles médicaux selon les revendications 1 à 3, caractérisés en ce que les indicateurs sont des colorants thermochromes signalant une stérilisation par de la vapeur d'eau.

7. Articles médicaux selon les revendications 1 à 3, caractérisés en ce que les indicateurs sont des colorants signalant une stérilisation par du gaz, par exemple des colorants qui réagissent avec l'oxyde d'éthylène.